# EUROPEAN PATENT APPLICATION

(11) **EP 2 511 375 A1**
(43) Date of publication of application: **17.10.2012**
(21) Application number: 11162196.7
(22) Date of filing: 13.04.2011
(51) Int. Cl.: C12P 7/00, C12P 7/06, C12P 7/56

(54) **High efficiency fermentation process**

(71) Applicant: Springhill S.A., 2522 Luxembourg (LU)
(72) Inventor: Ilushka, Igor Valerianovich, 141207, Pushkino (RU); Rakitin, Vladimir, 115419 Moscow (RU); Kolbakov, Vambola, 63202 Põlva parish (EE)
(74) Representative: Sarap, Margus

(57) **Abstract**

The present invention is related with a continuous periodic process for producing organic products such as ethanol, butanol, lactic acid, acetic acid and other base chemicals for further derivatizations. For fermentation are used carbohydrates containing materials, at the presence of cultures yeast or bacterial strain. The fermentation is carried out at conditions which provide the minimal cells growth rate of cultures yeast or bacterial strain in bioreactor and it results practically full conversion of carbohydrates.

## Description

### Technical Field

This invention relates to a method for producing organic products such as ethanol, butanol, lactic acid, acetic acid and others by continuous periodic fermentation of carbohydrates containing materials with a very high productivity and at practically full (up to 100%) conversion of carbohydrates.

### Background Art

The existing industries for the production of organic acids, alcohols and other products by fermentation are mostly based upon periodic batch operation, which usually carry out at low concentration of cultures yeast or bacterial strain (cells of microorganisms) within 60-80 hour.

When technologically optimal conversion of carbohydrates containing materials is reached, a fermentation stops and metabolites withdraw from the broth.

Such processes are characterized: by low productivity (low rate production of metabolites) and delivery of enormous amounts of waste water, which contain cells of microorganisms (i.e. stillage).

The amount of waste water can be a little reduced when fed-batch fermentation is used. Fed-batch fermentation is distinguished from batch fermentation by the addition, during operations, of a certain amount of fresh substrate and by the consequent withdrawal of a proportioned amount of broth. The fed-batch fermentation is characterized by a little longer overall time cycle that is certainly acceptable for industrial practice where at present very effective operating procedures for sterilization and the preventing of contamination are currently utilized.

But such process, as well as process of batch fermentation, is characterized by low productivity (low rate production of metabolites), and delivers enormous amounts of waste water, which contain cells of microorganisms.

One of the key points to increase the rate production of metabolites by fermentation is to maintain the microbial concentration as high as possible. At the same time, one way of reaching a high concentration of biomass in a fermenter and of decreasing of amounts of waste water, which contain cells of microorganisms, is the application of continuous cell-recycle bioreactors, in particular, cell-recycle membrane bioreactors.

These systems are mostly composed of continuous, well-stirred reactors either with a built-in membrane module or linked together with a separate membrane unit, most often operating on the basis of microfiltration or ultrafiltration.

However, the basic problem of the given continuous process is the incomplete conversion of carbohydrates. At that, the result of all attempts of increase a conversion of carbohydrates is decrease of productivity of fermentation.

It is necessary to note, that a water solution of metabolites, which is removed from a fermenter is free from cells of the microorganisms, but contains carbohydrates. The presence of relatively high amount of non-fermented carbohydrates in outlet of a fermenter essentially complicates process of isolation of target products, and also leads to increase in the cost of production.

Other of the serious problem of the membrane bioreactor is the difficulties connected with the deterioration of filtration rate in a long run of continuous fermentation because of incessant increase of biomass of cells of microorganisms.

For stabilization of filtration rate Asakura suggests to remove the microbial cake on the filter with periodic back flows of the fermentation gas through the filter elements. (T. Asakura and K. Toda, "New cell recycle ethanol fermentation with periodic cleaning of filter with gas" Bioprocess Engineering 7, 1991, 83-88). However, such actions move the process in the category of periodic processes and at the same time does not allow to achieve a high conversion of carbohydrates.

The purpose of the present invention is carrying out of fermentation process under such conditions at which: high productivity ensured, and practically full conversion of carbohydrates is reached.

### Summary of invention

According to present invention, organic products such as ethanol, butanol, lactic acid, acetic acid and other products can be produced in a simple and inexpensive manner by eliminating the disadvantages of the processes discussed above.

It has been surprisingly found, that if a process is carried out in periodic conditions at sufficiently high concentration of cells and in the conditions which provide very low growth rate of cells of microorganisms in fermenter, it is possible repeatedly to reuse cells of microorganisms isolated from a broth after each periodic stage of a fermentation on the following stage of a fermentation for conversion of a fresh party of carbohydrates.

It allows to carry out a fermentation with high rate (high productivity) and with practically full conversion of carbohydrates. This process essentially reduce both, the amount of wastewater and biomass, removed from a fermenter (so-called cell bleed) and, as consequence, to reduce a costs for its utilization.

It has been revealed, that for each microorganism there are optimal conditions - temperature, pH, amounts of nutrients and bacteriostatics fed into the fermenter, at which growth rate of cells is minimal but sufficient for replacement cells death. At the same time, the growth rate of cells can be reduced by increase of temperature and decrease of pH up to critical and supercritical values for each microorganism and as well by decrease of amounts nutrients fed into the fermenter and by use such of additives as bacteriostatics. Usually, the optimal growth rate of cells reach by combination of stirring and filtration speeds, temperature and pH rates, and by amount of needed amounts of nutrients and bacteriostatics fed into the fermenter.

It is necessary to note, that existing modern computer-controlled devices of measuring and control allow to automatize such a complicated periodic process completely.

### Description of embodiments

According to the present invention each periodic stage of production of organic products by fermentation comprises three steps:

1) loading a water solution of carbohydrates, nutrients, bacteriostatics, salts and cells of microorganisms to fermenter, concentration of cultures yeast or bacterial strain in a fermenter is maintained approximately on fixed state between 5-100 grams dry cell per litre (preferably 20-100 grams dry cell per litre);

2) fermentation with formation metabolites (organic products);

3) removal from fermenter a cell-free water solution metabolites, with re-direction to fermenter the concentrated in filter unit suspension of cells of microorganisms.

After that in fermenter, process containing with the concentrated suspension of cells (remained after the third stage), load a fresh portion of a water solution of carbohydrates, and if it is necessary, nutrients, bacteriostatics and salts, then repeat above operations (steps 2 and 3).

In spite of the fact that ten periodic stages are described in mentioned below examples, however the number of stages can be more than hundred and at that all stages are carried out without additives of fresh cells of microorganisms. This fact is an additional powerful costs saving point, as there is no need for the periodic cultivation of the inoculum (starting culture of fermentation, mostly 10% of fermentation volume).

It is necessary to note, that when periodic stages are repeated many times, nevertheless the small increase in concentration of cells of microorganisms takes place. Result of it is the increase in viscosity of a broth and reduction of rate of separation of cell from metabolites.

For elimination of this problem, periodically (after approximately 50-80 periodic stages have been carried out) a small amount of the concentrated suspension of cells of microorganisms is removed from fermenter to create concentration of cells in fermenter of approximately equal to initial concentration of cells (as at the first stage of fermentation).

The amount of the concentrated suspension of cells removed from a fermenter are essentially less in comparison with a stream of cells at continuous realization a fermentation (so-called "cell bleed"), not to mentioning about conventional periodic process of a batch-fermentation.

During the removing of the cell suspension, there are most of the non-soluble metabolites; part of cells lysis products etc is too removed from the process.

For carrying out of the given process a fermenter connected with a cell-recycle device can be used. Preferably, a cell-recycle device is a membrane module.

The given process allows to produce organic products with high productivity (up to 200 g/L/h) and in this conditions the amount of waste water, which contain cells of microorganisms, does not exceed values of 10 g per 1 kg of a target product.

For the preventing of the drastic loss of the fermentation yield after repeated cycles (so called resting), which depends at the microorganism used, there is possibly to add secondary culture to the fermentation broth. This culture added about in 3/4 process cycles carried out, and the amount added culture is up to 5% of the fermentation volume. During the continuing the fermentation, the main culture is suppressing the added one and the adding of the secondary culture can used again. Described action makes the usage of main culture 5-10 times longer and will have direct economic effect. The principle of phenomena by the data of publications is following - in nature microorganism exists as the mosaic films in the surfaces. They use each other metabolism and decay products like as symbionts. In artificial conditions, monoculture in water soluble stage, there is activated some still unknown mechanisms, preventing the uninhibited growth. The secondary culture has the role of compensation the artificial environment pressures.

The present invention is further illustrated in the following examples, which should not be taken to limit the scope of the invention.

Examples

The membrane recycle bioreactor system consists of the fermenter (10 L glass vessel) equipped with pH controller, controller of temperature and agitation. The fermenter was coupled in a semi-closed loop configuration to a membrane module (MW cut-off 100 kDa, F=1.8 M²), to separate cells of microorganism from the growth media and fermentation product.

Step 1.

At the beginning fermenter is load 5.7 I by mixture of the following composition, g/l:
Carbohydrates 30-200
Nutrients 3-6
Salts of K, Na, Mg and other 10-15
Bacteriostatics (optional) 1-6 (mg/l)
and suspension of cells of microorganisms for creation of concentration of cells in fermenter, equal to 5-100 g/l (counting upon dry-cells).

Step 2.

The process of fermentation is carried out up to practically full conversion of carbohydrates (>99.9%), automatically controlling temperature (temperature range of 37-55 °C) and pH (pH range of 5-7) in fermenter by the addition of 6-12% NH₄OH. When conversion of carbohydrates will achieve value of 99.9% the separation process is begins. In said step the minimal cells growth rate of cultures yeast or bacterial strain in bioreactor which is sufficient to provide cells death replacement is controlled by a choice of the certain temperature, pH and amount of nutrients feeding into the fermenter and by using of bacteriostatics.

Step 3.

The process of separation is carried out by passing under pressure the resulted broth via membrane module, collecting a water solution free of cell products and returning the concentrated suspension of cells to fermenter. Process of separation stop, when in a fermenter there will be approximately 10-15 % of a broth from its initial volume. The remained broth is concentrated suspension of cells of microorganisms.

The fresh solution of carbohydrates containing if it is necessary, nutrients, bacteriostatics and salts, is added to the remained broth and steps 2 and 3 repeat.

Glucose, lactose, fructose, galactose, sucrose can be used as carbohydrates. As nutrients peptone, protein extracts and yeast extract can be used. As salt K₂HPO₄, KH₂PO₄, NaCl, Na acetate, MgSO₄, Fe₂ (SO₄)₃, ZnSO₄, MnSO₄ and others can be used.

For the elongation of the fermentation (more filtration steps with one starting culture), there was added secondary culture of bacterium to the fermentation broth. The addition is carried out before a losing of the production efficiency of cultures. The best results were given with Lactococcus strain in combination with a Leuconostoc strain.

Ammonia used for the neutralization of fermentation environment utilized by the bacterium as the nitrogen source. Thus fewer nutrients needed. This makes the product cheaper and filtrate solution clearer. The last point makes the separation process easier, what means cheaper.

The fermentation method of the present invention can be applied not only to produce of lactic acid and ethanol, but also to the other organic acids, alcohols and esters, such as acetic acid, formic acid, propionic acid, citric acid, malic acid, maleic acid, malonic acid, fumaric acid, succinic acid, butanol, ethyl acetate and others.

Conditions and results of the fermentation are presented in tables 1, 2 and 3.

Table 1. The condition and results of fermentation of carbohydrates to lactic acid

**Table 1**

| Nº | Microorganism | Carbohydrate (C_{CH}¹) | Nutrients (C_{N}⁰) | Bacteriostatic (C_{B}) | Salts (C_{S}) | T | pH | C_{C}^{1 I} | C_{C}^{1 E} | C_{C}^{10E} | T_{F}¹ | T_{F}¹⁰ | C_{P} | G_{P}¹ | G_{P}¹⁰ | G_{P}^{A} |
|---|---|---|---|---|---|---|---|---|---|---|---|---|---|---|---|---|
| 1 | Lactococcus lactis 3905 | Glucose(50) | Peptone(4) | - | PP(4) | 39 | 33 | 7 | 8 | 14 | 65 | 61 | 44 | 28 | 30 | 29 |
| | | | | | SC (10) | | | | | | | | | | | |
| 2 | Lactococcus lactis 3905 | Lactose(100) | Yeast Extract(5) | - | PP(2) | 39 | 59 | 34 | 35 | 45 | 18 | 16 | 94 | 152 | 162 | 158 |
| | | | | | FS(2) | | | | | | | | | | | |
| | | | | | SC(8) | | | | | | | | | | | |
| 3 | Lactobacillus lactis 3123 | Glucose(30) | Yeast Extract(4) | - | PP(2) | 55 | 57 | 55 | 6 | 13 | 78 | 72 | 27 | 15 | 16 | 16 |
| | | | | | FS(0 1) | | | | | | | | | | | |
| | | | | | SC(9) | | | | | | | | | | | |
| 4 | Lactobacillus lactis 3123 | Lactose(90) | Peptone(4) | Erythro-mycin(2) | PP(4) | 38 | 58 | 28 | 30 | 42 | 35 | 31 | 86 | 90 | 97 | 95 |
| | | | | | SC(10) | | | | | | | | | | | |
| 5 | Lactobacillus casei 7657 | Sucrose(40) | Yeast Extract(4) | Tetra-cycline(1) | PP(3) | 43 | 49 | 8 | 9 | 17 | 71 | 66 | 38 | 23 | 24 | 24 |
| | | | | | SC(9) | | | | | | | | | | | |
| 6 | Lactobacillus casei 7657 | Sucrose(80) | Yeast Extract(5) | - | PP(4) | 49 | 39 | 30 | 32 | 40 | 22 | 18 | 77 | 110 | 124 | 119 |
| | | | | | SC(10) | | | | | | | | | | | |
| 7 | Lactococcus lactis 3905 | Sucrose(30) | Yeast Extract(10) | | PP(2) | 39 | 59 | 21 | 23 | 30 | 30 | 27 | 93 | 108 | 11B | 113 |
| | | Glucose(40) | | | FS(2) | | | | | | | | | | | |
| | | Lactose(30) | | | SC(8) | | | | | | | | | | | |
| 8* | Lactococcus lactis 3905 | Glucose(25) | Yeast Extract(10) | - | | 39 | 59 | 11 | 12 | 14 | 61 | 53 | 94 | 63 | 71 | 68 |
| | | Glucose(25) | | | PP(2) | | | | | | | | | | | |
| | | Glucose(25) | | | FS(2) SC(8) | | | | | | | | | | | |
| | | Glucose(25) | | | | | | | | | | | | | | |
| 9** | Lactococcus lactis 3905 Leuconostoc spp | Glucose(50) | Yeast Extract(5) | | PP(2) | 39 | 59 | | | | | | | | | |
| | | | | | FS(2) | | | | | | | | | | | |
| | | | | | SC(8) | | | | | | | | | | | |

| | | | | | | | | | | | | | | | | |
|---|---|---|---|---|---|---|---|---|---|---|---|---|---|---|---|---|
| Volume of fermentation (V_{F}) - 6 L, Conversion of carbohydrates >99 9%, To = 3 min; T_{S} = 10 min; V_{FT} = 5 L, * - all necessary quantity of carbohydrates has been broken on four parts and are added to fermenter during all period of fermentation every 15 minutes ** Fermentation example with the secondary bacterial culture, added in 10% fermenter volume at 85 stage of fermentation | | | | | | | | | | | | | | | | |

Table 2. The condition and results of fermentation of carbohydrates to ethanol

**Table 2**

| Nº | Microorganism | Carbohydrate (C_{CH}¹) | Nutrients (C_{N}⁰) | Bacteriostatic (C_{B}) | Salts (C_{S}) | T | pH | C_{C}^{1I} | C_{C}^{1E} | C_{C}^{10E} | T_{F}^{I} | T_{F}^{IU} | C_{P} | G_{P}^{I} | G_{P}^{IU} | G_{P}^{A} |
|---|---|---|---|---|---|---|---|---|---|---|---|---|---|---|---|---|
| 1 | Klyveromyces fragilis | Glucose(100) | Peptone(4) | Tetra- | PP(3) | 28 | 61 | 15 | 18 | 30 | 75 | 67 | 47 | 27 | 29 | 28 |
| | 2587 | | | cycline(1) | SC (9) | | | | | | | | | | | |
| 2 | Nyveromyces fragilis | Sucrose(200) | Yeast Extract(4) | Erythro- | PP(4) | 30 | 58 | 47 | 51 | 76 | 26 | 22 | 94 | 121 | 134 | 130 |
| | 2587 | | | mycin(1) | SC(8) | | | | | | | | | | | |
| 3 | Candida pseudotropicalis | Lactose(60) | Protein | - | PP(3) | 44 | 59 | 18 | 21 | 45 | 81 | 70 | 28 | 15 | 17 | 16 |
| | | | Extract(5) | | SC(9) | | | | | | | | | | | |
| 4 | Candida pseudotropicalis | Sucrose(120) | Yeast Extract(4) | - | PP(2) | 32 | 38 | 52 | 56 | 89 | 27 | 23 | 55 | 69 | 76 | 73 |
| | | | | | SC (9) | | | | | | | | | | | |

| | | | | | | | | | | | | | | | | |
|---|---|---|---|---|---|---|---|---|---|---|---|---|---|---|---|---|
| Volume of fermentation (VF) - 6 L; Conversion of carbohydrates >99.9%; To = 3 min; T_{S} = 10 min; V_{FT} = 5 L. | | | | | | | | | | | | | | | | |

Table 3. Ammonia Utilization by bacteria during the lactate fermentation

**Table 3**

| Nº | Microorganism | Carbohydrate (C_{CH}¹) | Nutrients (C_{N}⁰) | Salts (C_{S}) | T | pH | C_{C}^{1I} | C_{C}^{1E} | Neutralizator (C_{S}) | Neutralzator (C_{E}) | G _{P}^{A} |
|---|---|---|---|---|---|---|---|---|---|---|---|
| 1 | Lactococcus lactis 3905 | Glucose(50) | Yeast | PP(2) | 39 | 59 | 20 | 24 | Ammoma(4) | Ammoma(3 5) | 148 |
| | | | Extract(5) | FS(2) | | | | | | | |
| | | | | SC(8) | | | | | | | |
| | | | | | | | | | | | |
| 2 | Lactococcus lactis 3905 | Glucose(50) | Yeast | PP(2) | 39 | 59 | 20 | 21 | Ammonia(4) | Ammonia(3 2) | 134 |
| | | | Extract(1) | FS(2) | | | | | | | |
| | | | | SC(8) | | | | | | | |
| 3 | Lactococcus lactis 3905 | Glucose(50) | Yeast | PP(2) | 39 | 59 | 20 | 22 | NaOH(4) | NaOH(3 8) | 97 |
| | | | Extract(5) | FS(2) | | | | | | | |
| | | | | SC(8) | | | | | | | |

| | | | | | | | | | | | |
|---|---|---|---|---|---|---|---|---|---|---|---|
| Volume of fermentation (V_{F}) - 6 L; Conversion of carbohydrates >99.9%; To = 3 min; T_{S} = 10 min; V_{FT} = 5 L; carried 10 cycles. | | | | | | | | | | | |

### Notes in Table 1. 2.3.

T-the temperature of fermentation, °C:
C_{C}¹,-the initial concentration of cells of microorganisms in fermenter at the first stage of fermentation,
C_{C}^{1,E} - the concentration of cells of microorganisms in fermenter after the first stage of fermentation, gram/Litre (dry cell);
C_{C}^{10,E} - the concentration of cells of microorganisms in fermenter after the tenth stage of fermentation, gram/Litre (dry cell);
C_{CH}^{I} - the initial concentration of carbohydrates in fermenter at all stages of fermentation, gram/Litre;
C_{N}⁰ - the concentration of nutrients in fermenter at the first stage of fermentation, gram/Litre;
C_{S} - the concentration of salts in fermenter at all stages of fermentation, g/L;
C_{B} - the concentration of bacteriostatics in fermenter at all stages of fermentation, mg/L;
C_{S} - the concentration of neutralizator in the inlet, M
C_{E} - the concentration of neutralizator after the fermentation cycles (average), M
T₀ - the duration of batch charging of substrate, min; (its value approximately constant at all stages of fermentation and is approximately equal to 3 min)
T_{F}¹ - the duration of the first stage fermentation, min;
T_{F}¹⁰ - the duration of the tenth stage fermentation, min;
T_{S} - the duration of separation of products after fermentation, min. (Its value approximately constant at all stages of fermentation and is equal to 10± 2 min)
Cp - the concentration of product in filtrate, gram/Litre; (its value approximately constant after each stage of fermentation)
V_{FT} - the volume of filtrate at all stages of fermentation, L; (its value approximately constant after each stage of fermentation and is equal to 5 L)
G_{P}¹ - productivity of product on the first stage fermentation, g/(L*h);
G_{P}¹⁰ - productivity of product on the tenth stage fermentation, g/(L*h);
G_{P}^{A} - average productivity of product for ten stages of fermentation, g/(L*h);
PP - potassium phosphate;
MS - magnesium sulphate;
SF - ferric sulphate;
SM - salts of manganese;
SC - sodium chloride;
GP^{I} = (V_{FT} * C_{P})/ (T₀ + T_{F}ⁱ + T_{S})* V_{F}
G_{P}^{A} = Ó(V_{FT} * C_{P})/ (ΣT₀ + ΣT_{F}ⁱ + ΣT_{S})* V_{F}

## Claims

1. A high-efficiency continuous periodic process for production organic products by fermentation of carbohydrates containing materials comprising consecutive steps of:
a) microbiological fermentation of carbohydrates containing materials at the presence of cultures yeast or bacterial strain at conditions which provide:
i) the minimal cells growth rate of cultures yeast or bacterial strain in bioreactor but sufficient to provide cells death replacement.
ii) practically the full conversion of carbohydrates;
iii) simple and fast separation cells of microorganisms from metabolites;
b) separating of resulting biomass with generating of essentially cell-free aqueous solution of metabolites and of essentially metabolite-free concentrate of cells;
c) reuse of a concentrate of cells for carrying out of process of a fermentation of the fresh portion of carbohydrates containing materials;

2. The process according to claim 1, wherein the fermentation is carried out in a system membrane bioreactor which comprises a fermenter and a membrane separation module.

3. The process according to claim 1, wherein the minimal and sufficient cells growth rate of cultures yeast or bacterial strain is provided by a choice of the certain temperature, pH, amount of nutrients feeding into the fermenter and by using of bacteriostatics.

4. The process according to claim 3, wherein the temperature is in the range of 37-55 C° and the pH in the range of 5-7.

5. The process according to claim 1, wherein concentration of cultures yeast or bacterial strain in a fermenter is maintained approximately on fixed state between 5-100 grams dry cell per litre, preferably 20-100 grams dry cell per litre.

6. The process according to claim 1, wherein using the starches as carbohydrates source, used simultaneous saccharification and fermentation (SSF) process.

7. The process according to claim 1, wherein for the longevity of the continuous process, there is added secondary culture to the fermentation process.

8. The process according to the claim 6, wherein the secondary culture is added about in 3/4 process cycles carried out and the amount of added second culture is up to 5% of the fermentation volume.

9. The process according to claim 1, wherein by needs for neutralization (organic acids production) the preferred one is ammonia, as it possesses the nitrate source habit.
